# EUROPÄISCHE PATENTANMELDUNG

(11) **EP 0 797 983 A2**
(43) Veröffentlichungstag der Anmeldung: **01.10.1997**
(21) Anmeldenummer: 97104486.2
(22) Anmeldetag: 17.03.1997
(51) Int. Cl.: A61K 7/48, A61K 31/075, A61K 47/08

(54) **Verwendung von Methylethern in kosmetischen und/oder pharamazeutischen Zubereitungen**

(30) Priorität: 25.03.1996 DE 19611624
(71) Anmelder: Henkel Kommanditgesellschaft auf Aktien, 40589 Düsseldorf-Holthausen (DE)
(72) Erfinder: Trius, Antonio, Dr., Valldoreix Sant Cugat (ES); Pi Subirana, Rafael, Dr., 08400 Granollers (ES); Prat Queralt, Ester, Dr., 08238 Calella (ES); Bigorra Llosas, Joaquim, Dr., 08203 Sabadell (ES)

(57) **Zusammenfassung**

Methylether der Formel **(I)**, 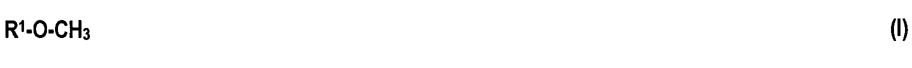 in der R¹ für einen aliphatischen, linearen oder verzweigten Alkylrest mit 6 bis 22 Kohlenstoffatomen und 0 und/oder 1, 2 oder 3 Doppelbindungen steht, eignen sich als Ölkörper für die Herstellung von kosmetischen und/oder pharmazeutischen Zubereitungen.

## Beschreibung

### Gebiet der Erfindung

Die Erfindung betrifft die Verwendung von ausgewählten Methylethern als Ölkomponenten für die Herstellung von kosmetischen bzw. pharmazeutischen Mitteln.

### Stand der Technik

Die weitaus meistens kosmetischen bzw. pharmazeutischen Zubereitungen, wie beispielsweise Cremes, Lotionen oder Salben, stellen Emulsionen von Ölkörpern in Wasser dar. Die Ölkörper stellen in diesen Zubereitungen gewöhnlich die wirkenden Komponenten dar, d.h. sie sind beispielsweise für pflegende oder fettende Eigenschaffen verantwortlich. Man unterscheidet zwischen hoch spreitenden, polaren und niedrig spreitenden, unpolaren Ölen. Bei ersteren, zu denen beispielsweise Pflanzenöle zählen, handelt es sich um Stoffe, die sich zwar sehr rasch auf der Haut verteilen, deren Wirkung jedoch nicht lange anhält. Letztere, zu denen beispielsweise die Dioctylether gehören, vermitteln zwar ein lang andauerndes Hautgefühl, verteilen sich jedoch nur sehr langsam auf der Hautoberfläche. Da keine der beiden Gruppen alleine sowohl eine rasch einsetzende als auch lang anhaltende Wirkung entfaltet, werden in Cremes und Lotionen üblicherweise Mischungen von Ölkörpern unterschiedlicher Spreitwerte eingesetzt ( 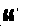 Spreitkaskade 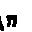 ). Eine Übersicht zu diesem Thema findet sich beispielsweise von S.Wallat in **Parf. Kosm. 75, 768 (1994)**.

Zieht man die große Zahl geeigneter Ölkörper und deren unterschiedlichsten Eigenschaften in Betracht, wird sofort klar, daß für die Entwicklung einer nach ihrer hautkosmetischen Wirkung optimierten Formulierung eine Vielzahl von Versuchen erforderlich ist. Nachdem sich das Spreitverhalten und das resultierende Hautgefühl einer Mischung von verschiedenen Ölkörpern nicht vorhersagen läßt, besteht in der Fachwelt ein lebhaftes Interesse an Möglichkeiten, die Zahl erforderlicher Experimente bei der Rezepturentwicklung zu vermindern. Insbesondere besteht ein Bedürfnis an neuen Ölkörpern, die sich universeller einsetzen lassen als bekannte Stoffe.

Symmetrische Dialkylether, wie beispielsweise Dioctylether, stellen niedrig spreitende Ölkörper dar, die der Haut zwar ein lang anhaltendes Glättegefühl vermitteln, sich wegen ihrer geringen Polarität jedoch häufig nur mit Schwierigkeiten formulieren lassen. So zeigen Formulierungen mit symmetrischen Dialkylethern die Tendenz, im Laufe der Lagerung ihre Viskosität zu verändern und sich gegebenenfalls sogar zu entmischen. Die Aufgabe der Erfindung hat nun darin bestanden, neue Ölkörper zur Verfügung zu stellen, die frei von den geschilderten Nachteilen sind und insbesondere bei vergleichbarer hautkosmetischer Wirkung eine vereinfachte Einarbeitung in kosmetische bzw. pharmazeutische Zubereitungen gestatten.

### Beschreibung der Erfindung

Gegenstand der Erfindung ist die Verwendung von Methylethern der Formel **(I)**, 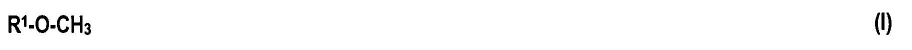 in der R¹ für einen aliphatischen, linearen oder verzweigten Alkylrest mit 6 bis 22 Kohlenstoffatomen und 0 und/oder 1, 2 oder 3 Doppelbindungen steht, als Ölkörper für die Herstellung von kosmetischen und/oder pharmazeutischen Zubereitungen.

Überraschenderweise wurde gefunden, daß die unsymmetrischen Methylether der Formel (I) gegenüber bekannten Ölkörpern vom Typ der symmetrischen Di-n- bzw. Di-i-octylether trotz höheren Spreitvermögens auf der Haut ein gleichmäßigeres und länger andauerndes Glättegefühl bewirken. Die Stoffe lassen sich problemlos in Emulsionen einarbeiten, die resultierenden Emulsionen zeigen zudem eine signifikant verbesserte Lagerstabilität.

### Methylether

Die im Sinne der Erfindung zu verwendenden Methylether stellen bekannte Stoffe dar, die nach den einschlägigen Verfahren der präparativen organischen Chemie erhalten werden können. Ein Verfahren zu ihrer Herstellung besteht darin, die entsprechenden Alkohole in Gegenwart starker Basen in ihre Alkoholate zu überführen und diese dann mit Dimethylsulfat umzusetzen. Die bei der Methylierung als Nebenprodukte gebildeten Alkalimethylsulfat-Salze können anschließend ausgewaschen werden.

Geeignete Alkohole für die Herstellung der Methylether stellen beispielsweise die folgenden dar: Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Aus anwendungstechnischer Sicht sind Methylether auf Basis von Kokosfettalkoholen mit 12 bis 14 bzw. Cetearylalkoholen mit 16 bis 18 Kohlenstoffatomen bevorzugt.

### Methylierung

Als Basen zur Herstellung der Alkoholate kommen insbesondere Alkalihydroxide, vorzugsweise Natrium-und/oder Kaliumhydroxid in Frage, die in Mengen von 100 bis 400 und vorzugsweise 150 bis 250 Mol-% - bezogen auf die Alkohole - eingesetzt werden. Es hat sich weiterhin als vorteilhaft erwiesen, die Alkoholatbildung in Gegenwart von 0,01 bis 0,1 und vorzugsweise 0,02 bis 0,05 Gew.-% - bezogen auf die Alkohole - Alkaliborhydriden, vorzugsweise Natriumborhydrid, durchzuführen. Die Temperatur für die Herstellung der Alkoholate sollte im Bereich von 40 bis 80°C liegen, während die Methylierung unter Zugabe von Dimethylsulfat üblicherweise eine Temperatur von 60 bis 70°C nicht übersteigen sollte. Das molare Einsatzverhältnis Alkohol zu Dimethylsulfat liegt in der Regel bei 1 : 0,95 bis 1 : 1,5 und vorzugsweise im Bereich von 1 : 1 bis 1 : 1,3. Im Anschluß an die Methylierung empfiehlt es sich, die Reaktionsmischung einer Nachreaktion bei 70 bis 80°C zu unterwerfen. Die rohen Ether werden dann in der Regel gewaschen, mit Wasser versetzt und auf 80 bis 85°C erhitzt, um nicht abreagiertes DMS zu zerstören. Anschließend wird die organische Wertphase abgetrennt und dann getrocknet.

### Gewerbliche Anwendbarkeit

Die erfindungsgemäßen Methylether besitzen ein vorteilhaftes Spreitvermögen und vermitteln der Haut ein gleichmäßiges und langandauerndes Glättegefühl. Sie eignen sich daher zur Herstellung von kosmetischen und/oder pharmazeutischen Zubereitungen, wie beispielsweise Tagescremes, Nachtcremes, Pflegecremes, Nährcreme, Bodylotions, Salben und dergleichen. Diese Mittel können als Hilfs-und Zusatzstoffe weitere Ölkörper, Emulgatoren, Überfettungsmittel, Fette, Wachse, Stabilisatoren, biogene Wirkstoffe, Konservierungsmittel, Farb- und Duftstoffe und dergleichen enthalten.

Als zusätzliche **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimerdiol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate, Dioctylether und/oder aliphatische bzw. naphthenische Kohlenwasserstoffe in Betracht.

Als **Emulgatoren bzw. Co-Emulgatoren** können nichtionogene, ampholytische und/oder zwitterionische grenzflächenaktive Verbindungen verwendet werden, die sich durch eine lipophile, bevorzugt lineare Alkyl- oder Alkenylgruppe und mindestens eine hydrophile Gruppe auszeichnen. Diese hydrophile Gruppe kann sowohl eine ionogene als auch eine nichtionogene Gruppe sein. Nichtionogene Emulgatoren enthalten als hydrophile Gruppe z. B. eine Polyolgruppe, eine Polyalkylenglycolethergruppe oder eine Kombination aus Polyol- und Polyglycolethergruppe.

Bevorzugt sind solche Mittel, die als **O/W-Emulgatoren** nichtionogene Tenside aus mindestens einer der folgenden Gruppen enthalten:
(a1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(a2) C_{12/18}-Fettsäuremono- und -diester von Anlagerungsprodukten von 1 bis 30 Mol Ethylenoxid an Glycerin;
(a3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(a4) Alkylmono- und -oligoglycoside mit 8 bis 22 Kohlenstoffatomen im Alkylrest und deren ethoxylierte Analoga;
(a5) Anlagerungsprodukte von 15 bis 60 Mol Ethylenoxid an Ricinusöl und/oder gehärtetes Ricinusöl;
(a6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18} - Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und oligoglycoside, ihre Herstellung und ihre Verwendung als oberflächenaktive Stoffe sind beispielsweise aus **US 3,839,318, US 3,707,535, US 3,547,828, DE-OS 19 43 689, DE-OS 20 36 472** und **DE-A1 30 01 064** sowie **EP-A 0 077 167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl* Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder - SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Als **W/O-Emulgatoren** kommen in Betracht:
(b1) Anlagerungsprodukte von 2 bis 15 Mol Ethylenoxid an Ricinusöl und/oder gehärtetes Ricinusöl;
(b2) Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose);
(b3) Trialkylphosphate;
(b4) Wollwachsalkohole;
(b5) Polysiloxan-Polyalkyl-Polyether-Copolymere bzw. entsprechende Derivate;
(b6) Mischester aus Pentaerythrit, Fettsäuren, Citronensäure und Fettalkohol gemäß **DE-PS 11 65 574** sowie
(b7) Polyalkylenglycole.

Als **Überfettungsmittel** können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdikkungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationischen Cellulosederivate, kationischen Stärke, Copolymere von Diallylammoniumsalzen und Acrylamiden, quaternierte Vinylpyrrolidon/Vinylimidazol-Polymere wie z.B. Luviquat® (BASF AG, Ludwigshafen/ FRG), Kondensationsprodukte von Polyglycolen und Aminen, quaternierte Kollagenpolypeptide wie beispielsweise Lauryldimonium hydroxypropyl hydrolyzed collagen (Lamequat®L, Grünau GmbH), quaternierte Weizenpolypeptide, Polyethylenimin, kationische Siliconpolymere wie z.B. Amidomethicone oder Dow Corning, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der **FR-A 22 52 840** sowie deren vernetzte wasserlöslichen Polymere, kationische Chitinderivate wie beispielsweise quaterniertes Chitosan, gegebenenfalls mikrokristallin verteilt, kationischer Guar-Gum wie z.B. Jaguar® CBS, Jaguar® C-17, Jaguar® C-16 der Celanese/US, quaternierte Ammoniumsalz-Polymere wie z.B. Mirapol® A-15, Mirapol® AD-1, Mirapol® AZ-1 der Miranol/US.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen. Unter **biogenen Wirkstoffen** sind beispielsweise Pflanzenextrakte und Vitaminkomplexe zu verstehen. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als **Konservierungsmittel** eignen sich beispielsweise Phenoxyethanol, Formaldehydlösung, Parabene, Pentandiol oder Sorbinsäure. Als **Perlglanzmittel** kommen beispielsweise Glycoldistearinsäureester wie Ethylenglycoldistearat, aber auch Fettsäuremonoglycolester in Betracht. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen.

### Beispiele

### Beispiel 1

In einem 2-l-Dreihalskolben mit Rührer und Tropftrichter wurden 400 g (1,56 mol) Cetearylalkohol, 4 g (0,06 mol) Kaliumhydroxidschuppen (87 Gew.-%ig) und 0,24 g Natriumborhydrid vorgelegt. Die Mischung wurde auf 60°C erwärmt und über einen Zeitraum von 30 min portionsweise mit weiteren 186 g (2,88 mol) Kaliumhydroxid versetzt. Anschließend wurde die Reaktionsmischung 30 min gerührt und dann über den Tropftrichter portionsweise 246 g (1,96 mol) Dimethylsulfat zugesetzt, wobei die Temperatur unterhalb von 70°C gehalten wurde. Danach wurde die Mischung zunächst 120 min bei 60°C und dann 60 min bei 80°C gerührt, schließlich mit 1100 ml Wasser und dann mit 130 g (1,16 mol) wäßriger 50 Gew.-%iger Kalilauge weitere 120 min bei 80 bis 85°C gerührt. Anschließend wurde die wäßrige Phase abgetrennt, die organische Phase getrocknet und filtriert. Der Cetearylmethylether wurde in einer Ausbeute von 88 % der Theorie erhalten.

### Beispiel 2

In einem 2-l-Dreihalskolben mit Rührer und Tropftrichter wurden 350 g (1,8 mol) Kokosalkohol, 4 g (0,06 mol) Kaliumhydroxidschuppen und 0,24 g Natriumborhydrid vorgelegt. Die Mischung wurde auf 45°C erwärmt und über einen Zeitraum von 30 min portionsweise mit weiteren 214 g (3,32 mol) Kaliumhydroxid versetzt. Anschließend wurde die Mischung 30 min gerührt und über den Tropftrichter portionsweise 284 g (2,25 mol) Dimethylsulfat zugesetzt, wobei die Temperatur unterhalb von 60°C gehalten wurde. Danach wurde die Mischung zunächst 120 min bei 50°C und dann 60 min bei 80°C gerührt, schließlich mit 1300 ml Wasser und dann mit 152 g (1,35 mol) wäßriger 50 Gew.-%iger Kalilauge weitere 120 min bei 80 bis 85°C gerührt. Anschließend wurde die wäßrige Phase abgetrennt, die organische Phase getrocknet und filtriert. Der Kokosalkylmethylether wurde in einer Ausbeute von 89 % der Theorie erhalten.

### Beispiele 3 und 4

In der nachfolgenden Tabelle 1 finden sich Formulierungsbeispiele für eine niedrigviskose Lotion und eine höher viskose Creme. Die Herstellung der beiden Formulierungen erfolgte durch Vermischen der Einsatzstoffe bei 80°C. Die Formulierungen zeigten bei Lagerung (6 Wochen, 40°C) eine konstante Viskosität und keine Entmischung. Analoge Formulierungen, die anstelle des Cetearylmethylethers Di-n-octylether als Ölkörper enthielten, zeigten eine im Laufe der Lagerung abnehmende Viskosität; die Vergleichslotion entmischte sich nach 4 Wochen. Auch die sensorische Beurteilung der Vergleichsformulierungen war deutlich schlechter.

**Tabelle 1**

| **Formulierungsbeispiele** | | |
|---|---|---|
| **Bestandteil** | **Lotion [Gew.-%]** | **Creme [Gew.-%]** |
| Cetearylglucosid | 1 | 2 |
| Cetearylalkohol | 3 | 6 |
| Cocoamidopropyl Betaine | 0,5 | 0,5 |
| Cetearylmethylether | 35 | 35 |
| Wasser | ad 100 Gew.-% | |

## Patentansprüche

1. Verwendung von Methylethern der Formel **(I)**, 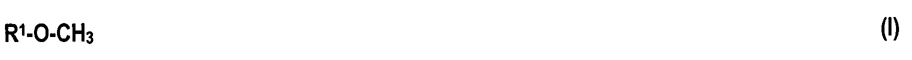 in der R¹ für einen aliphatischen, linearen oder verzweigten Alkylrest mit 6 bis 22 Kohlenstoffatomen und 0 und/oder 1, 2 oder 3 Doppelbindungen steht, als Ölkörper für die Herstellung von kosmetischen und/oder pharmazeutischen Zubereitungen.

2. Verwendung nach Anspruch 1, **dadurch gekennzeichnet**, daß man Methylether der Formel **(I)** einsetzt, in der R¹ für einen linearen Alkylrest mit 12 bis 14 Kohlenstoffatomen steht.

3. Verwendung nach Anspruch 1, **dadurch gekennzeichnet**, daß man Methylether der Formel **(I)** einsetzt, in der R¹ für einen linearen Alkylrest mit 16 bis 18 Kohlenstoffatomen steht.
